Europäisches Patentamt
European Patent Office
Office européen des brevets

(19)

(11) Publication number: **0 252 404 A2**

(12) **EUROPEAN PATENT APPLICATION**

(21) Application number: 87109355.5

(22) Date of filing: 30.06.87

(51) Int. Cl.$^3$: C 07 D 209/18
C 07 D 493/10, G 01 N 33/5-33
G 01 N 33/68, G 01 N 33/542
//(C07D493/10, 311:00, 307:00)

(30) Priority: 09.07.86 US 883883

(43) Date of publication of application: 13.01.88 Bulletin 88/2

(84) Designated Contracting States: AT BE CH DE ES FR GB IT LI NL

(71) Applicant: ABBOTT LABORATORIES
14th Street and Sheridan Road North St
North Chicago Illinois 60064(US)

(72) Inventor: Adamczyk, Maciej Bogdan
2015 Sprucewood Lane
Lindenhurst Illinois 60046(US)

(72) Inventor: Flentge, Charles Arthur
25296 W. Wayside Place
Lake Villa Illinois 60046(US)

(72) Inventor: Brashear, Roy Jeffrey
145 Sylvan Drive North
Mundelein Illinois 60060(US)

(74) Representative: Modiano, Guido et al,
MODIANO, JOSIF, PISANTY & STAUB Modiano & Associati Via Meravigli, 16
I-20123 Milan(IT)

(54) **5-Hydroxy-3-indoleacetic acid fluorescence polarization immunoassay.**

(57) The present invention is directed to a fluorescence polarization immunoassay for determining the 5-hydroxy-3-indoleacetic acid content in body fluids, to the various components needed for preparing and carrying out such an assay, and to methods of making these components. Specifically, tracers, immunogens and antibodies are disclosed, as well as methods for preparing them. The assay is conducted by measuring the degree of polarization of plane polarized light that has been passed through a sample containing antiserum and tracer.

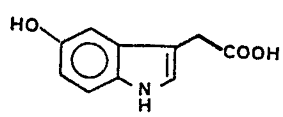

FIG. 1

EP 0 252 404 A2

11·30·06·87



# 5-HYDROXY-3-INDOLEACETIC ACID FLUORESCENCE POLARIZATION IMMUNOASSAY

## BACKGROUND OF THE INVENTION

### Technical Field

The present invention relates to a method and reagents for fluorescence polarization immunoassay procedure for determining the amount of 5-hydroxy -3-indoleacetic acid (5-HIAA) in fluids, especially biological fluids such as serum, plasma or urine, and to a method of making the reagents. More specifically, the invention relates to (1) reagents (tracers and antibodies) for determining the amount of 5HIAA in a sample; (2) immunogen compounds used to raise antibodies; (3) synthetic methods (for making the tracer and immunogen compounds); and (4) analytical methods for conducting the assay.

### Background Art

5-HIAA is the principal metabolite of the neurotransmitter serotonin (5-hydroxytryptamine). Measurements of urinary 5-HIAA levels have been used as a convenient indicator of serotonergic dysfunction in the body. Moderately to highly elevated levels of 5-HIAA have been associated both with malabsorption

syndrome and with a second syndrome characterized by metabolic and neurologic disturbances which may lead to the occurrence of extremely serious right-sided valvular heart disease.

Gas chromatography with mass spectrometry, high performance liquid chromatography, radioimmunoassay and colorimetric methods have been described in the literature for 5-HIAA determination in urine. In general, competitive binding immunoassays have provided a preferable alternative to chemical methods such as gas chromatography and high pressure liquid chromatography, which require lengthy sample extraction procedures and assay times. This is true of the fluorescence polarization immunoassay of the present invention, which combines the specificity of an immunoassay with the speed and convenience of a homogeneous method to offer a precise and reliable procedure for monitoring 5-HIAA levels in urine.

In a competitive binding immunoassay, the biological substance being measured (usually referred to as the "ligand") competes with a labeled reagent (the "ligand analog" or "tracer") for a limited number of receptor binding sites on antibodies specific to the ligand and ligand analog. The concentration of ligand in the sample determines the amount of ligand analog which binds to the antibody. The amount of ligand analog that will bind is inversely proportional to the concentration of ligand in the sample, because the ligand and the ligand analog each bind to the antibody in proportion to their respective concentrations.

Fluorescence polarization is one method that may be utilized to measure the amount of tracer-antibody conjugate produced in a competitive binding immunoassay. Fluorescence polarization techniques are based on the principle that a fluorescent labeled

compound, when excited by plane polarized light, will emit fluorescence having a degree of polarization inversely related to its rate of rotation. Accordingly, when a tracer-antibody conjugate having a fluorescent label is excited with plane polarized light, the emitted light remains highly polarized because the fluorophore is constrained from rotating between the time that light is absorbed and emitted. In contrast, when an unbound tracer is excited by plane polarized light, its rotation is much faster than that of the corresponding tracer-antibody conjugate. As a result, the light emitted from the unbound tracer molecules is depolarized.

Fluorescence polarization immunoassay techniques are well known in the art; to date, however, the use of such techniques for the determination of urinary 5-HIAA levels has not been successfully attempted. The present invention offers an advance in the art in that highly accurate determinations of the amount of 5-HIAA in urine are obtainable with increased convenience, at decreased expense, without the disadvantages heretofore imposed by the various available assay systems.

SUMMARY OF THE INVENTION

The present invention is directed to a fluorescence polarization assay for 5-HIAA to tracers, immunogens and antibodies for use in the assay; and to methods for making the tracers, immunogens and antibodies.

A first aspect of the invention relates to the discovery of unique tracers and immunogens having novel structures. According to the first aspect of the invention, the tracers and the immunogens can both be represented by the structural formula shown in Figure 2 of the drawings wherein:

$R_1$ is H or R-Z-Q or together with $R_2$ is a cyclic group having a total of from 4 to 8 carbon atoms and containing up to 4 heteroatoms;

$R_2$ is H, OH, Cl, F, Br, I, $OCH_3$, O-R-Z-Q, or together with $R_1$ or $R_3$ is a cyclic group having a total of from 4 to 8 carbon atoms and containing up to 4 heteroatoms;

$R_3$ is H or R-Z-Q or together with $R_2$ is a cyclic group having a total of from 4 to 8 carbon atoms and containing up to 4 heteroatoms;

$R_4$ is H or R-Z-Q;

Q is a poly(amino acid), a poly(amino acid) derivative or another immunologically active carrier, or fluorescein or a fluorescein derivative;

Z is CO, NH, $CH_2NH$ or CS when Q is fluorescein or a fluorescein derivative and is N, NH, $SO_2$, $PO_2$, PSO or a glucuronide moiety when Q is a poly(amino acid), poly(amino acid) derivative or other immunologically active carrier; and

R is a linking group including up to 7 heteroatoms when Q is a poly(amino acid), poly(amino acid) derivative or other immunologically active carrier, including up to 10 heteroatoms when Q is fluorescein or a fluorescein derivative, and having a total of from 0 to 20 carbon atoms and heteroatoms arranged in a straight or branched chain and containing up to two ring structures.

When Q is a poly(amino acid), a derivative thereof or any other immunologically active carrier, the compound can be used as an immunogen. When Q is fluorescein or a derivative thereof, the compound can be used as a tracer.

In a presently preferred embodiment of the invention, the tracers and immunogens can both be represented by the structural formula shown in Figure 3, wherein:

$R_2$ is H, OH, Cl, F, Br, or I;
$R_4$ is R-Z-Q;
Q and Z are as previously defined; and
R is a linking group including up to 4 heteroatoms when Q is a poly(amino acid), a poly(amino acid) derivative or other immunologically active carrier and including up to 10 heteroatoms when Q is fluorescein or a fluorescein derivative, and having a total of from 0 to 20 carbon atoms and heteroatoms arranged in a straight or branched chain and containing up to two ring structures.

A second aspect of the invention relates to antibodies raised by the novel immunogens. According to the second aspect of the invention, antibodies are prepared in response to a compound according to Claim 1 wherein Q is a poly(amino acid), a poly(amino acid) derivative or another immunologically active carrier.

According to a third aspect of the invention, an immunogen is made by a method comprising the step of coupling (a) a compound represented by the structural formula shown in Figure 2 wherein Z is $NH_2$, $CO_2H$, or $NH-COCH_2I$, and R is a linking group having a total of from 0 to 20 carbon atoms and heteroatoms arranged in a straight or branched chain and containing up to two ring structures, with (b) a poly(amino acid), a poly(amino acid) derivative or another immunologically active carrier. Preferred poly(amino acids) include thyroglobulin and keyhole limpet hemocyanin.

According to a fourth aspect of the invention, a method is provided for making a tracer by coupling (a) a compound represented by the structural formula shown in Figure 2 wherein ZQ is $CO_2H$, $NH_2$, OH, $SO_3H$ or CHO, and R is a linking group having a total of from 0 to 20 carbon atoms and heteroatoms arranged in a straight or branched chain and containing up to two ring

structures, with (b) fluorescein or a derivative of fluorescein. Presently preferred are the derivatives of 4'-aminomethylfluorescein.

A fifth aspect of the invention relates to a novel stabilizing matrix for the tracers of the invention. Such stabilizing matrix comprises an organic solvent having a pH in the range of 1.5 to 3.5, an antioxidant that is stable and soluble in such organic solvent, and an electron-conductive compound. One such matrix comprises N,N-dimethylformamide as the organic solvent, thiourea as an antioxidant, and potassium chloride/HCl as the electron-conductive compound.

A sixth aspect of the invention relates to the novel use of a specimen preparation method utilizing a phase extraction for reduction of specimen background and cross-reacting metabolites.

A seventh aspect of the invention relates to a novel stabilizing matrix for the controls and calibrators of the invention. This matrix comprises an artificial urine matrix containing certain normally occurring components of urine plus a combination of L-ascorbic acid and N-acetyl-L-cysteine, the matrix having been purged of oxygen and sealed.

According to a ninth aspect of the invention, a process for measuring the concentration of 5-HIAA is provided. A sample is contacted with 5-HIAA antiserum, a background quenching compound, and a fluorescein-containing 5-HIAA derivative capable of producing a detectable fluorescence polarization response to the presence of the 5-HIAA antiserum. Plane-polarized light is then passed through the solution to obtain a fluorescence polarization response, and this response is detected as a measure of the amount of 5-HIAA in the sample. The improved process of this aspect of the invention allows for greatly increased

specimen throughput as compared to such methods of the prior art as gas chromatography with mass spectrometry, high performance liquid chromatography, radioimmunoassay and colorimetric methods.

Further objects and attendant advantages of the invention will be best understood from a reading of the following detailed description taken together with the figures and the examples.

BRIEF DESCRIPTION OF THE DRAWINGS

In the following figures the symbol "Fl" represents a fluorescein moiety, "BSA" represents bovine serum albumin, "KLH" represents keyhole limpet hemocyanin, and "TGB" represents thyroglobulin.

Figure 1 shows the structure of the metabolite 5-HIAA to be quantitatively determined in accordance with the present invention.

Figure 2 shows a general structural formula for the tracers and the immunogens of the present invention as well as the classes of reactants used in preparing them.

Figure 3 shows a general structural formula for a presently preferred class of tracers and immunogens;

Figure 4 shows the alternate structural formulae and names of the fluorescein moiety included in the tracers of the present invention.

Figure 5 shows various linkages that couple the fluorescein moiety to the precursor in Figure 2 when Figure 2 represents a precursor for the tracers.

Figures 6 through 9 and Figure 30 show various examples of structurers of tracers in accordance with the present invention.

Figures 10, 11 and 25 show examples of immunogens in accordance with the present invention.

Figures 12 through 24 and 26 through 29 show various examples of structures of hapten reactants used to form the tracers and immunogens employed in the present invention.

DETAILED DESCRIPTION OF THE INVENTION

The present invention involves the use of fluorescein and derivatives of fluorescein. A necessary property of fluorescein and its derivatives for the usefulness of the tracer compounds herein is the fluorescence of fluorescein. Fluorescein exists in two tautomeric forms, illustrated in Figure 3, depending on the acid concentration (pH) of the environment. In the open (acid) form, there are a number of conjugated double bonds which make that form of fluorescein (and compounds containing a fluorescein moiety) capable of absorbing blue light and emitting green fluorescence after an excited state lifetime of about four nanoseconds. When the open and closed forms coexist, the relative concentration of molecules in the open and closed forms is easily altered by adjustment of the pH level. Generally, the tracer compounds of the present invention exist in solution as biologically acceptable salts such as sodium, potassium, ammonium and the like, which allows the compounds to exist in the open, fluorescent form, when employed in the analytical methods of the present invention. The specific salt present will depend on the buffer employed to adjust the pH level. For example, in the presence of a sodium phosphate buffer, the compounds of the present invention will generally exist in the open form, as a sodium salt.

As used herein, the term "fluorescein," either as an individual compound or as a component of a larger compound, is meant to include both the open and closed forms, if they exist for a particular molecule, except

in the context of fluorescence. An open form is necessary for the fluorescence to occur.

The numbering of carbon atoms of the fluorescein molecule varies, depending upon whether the open or closed form of the molecule is considered. Accordingly, the literature concerning fluorescein and its compounds is not uniform as to carbon atom numbering. In the closed form, the para-carbon to the carbonyl of the lactone on the phenyl ring is numbered 6. In the open form, the para-carbon to the carboxylic acid group on the phenyl ring is numbered 5 (see Figure 4). In this disclosure, the numbering of the closed form is adopted because the raw materials used in the syntheses are most popularly numbered with that system. The carbon atom of fluorescein and its compounds which is opposite the carboxyl group is therefore numbered "6" for the purposes of the present disclosure.

A tracer in solution which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule which, because of its larger space, is slower than that of the relatively small tracer molecule, thereby increasing the polarization observed. Therefore, when a ligand competes with the tracer for antibody sites, the observed polarization of fluorescence of the resulting mixture of the free tracer and tracer-antibody complex assumes a value intermediate between that of the tracer and that of the tracer-antibody complex. If a sample contains a high concentration of the ligand, the

observed polarization value is closer to that of the free ligand, i.e., low. If the test sample contains a low concentration of the ligand, the polarization value is closer to that of the bound ligand, i.e., high. By sequentially exciting the reaction mixture of an immunoassay with vertically and then horizontally polarized light and analyzing only the vertically polarized component of the emitted light, the polarization of fluorescence in the reaction mixture may be accurately determined. The precise relationship between polarization and concentration of the ligand to be determined is established by measuring the polarization values of calibrators with known concentrations. The concentration of the ligand can be interpolated from a standard curve prepared in this manner.

The Reagents

Both the immunogens and the tracers of the present invention can be represented by the general structural formula illustrated in Figure 2.

The objective is to have competition between 5-HIAA and the tracer for the recognition sites of the antibody. Great variations in the structure of the haptens and tracers are allowed in achieving this goal. For purposes of this invention, "haptens" are precursors of the immunogens, comprising generally a substituted 5-HIAA derivative bearing a group suitable for linking to an immunologically active carrier.

The Structure of the Immunogens

Usable antibodies can be produced from a variety of 5-HIAA derivatives. Immunogens made from compounds functionalized at the 1-position, 4-position, 5-position and 6-position can produce antibodies in

animals; such antibodies are useful in an 5-HIAA assay according to the invention when combined with the appropriate tracer.

The immunogens of the present invention have the general structural formula shown in Figure 2, and in the preferred form of the invention, the immunogens are also derived from the general structural formula shown in Figure 3. The immunogens can be prepared by coupling a compound of the class shown in Figure 2 with a poly(amino acid) or a derivative of a poly(amino acid) or other immunologically active carrier as will be discussed in the context of the synthetic method and the Examples below. Although TGB and KLH are the poly(amino acid) in this preferred form, it should be understood that various protein carriers can be employed, including albumins, serum proteins, e.g., globulins, ocular lens proteins, lipoproteins and the like. Illustrative protein carriers include bovine serum albumin, egg ovalbumin, bovine gamma-globulin, thyroxine binding globulin, etc. Alternatively, synthetic poly(amino acids) having a sufficient number of available carboxylate groups such as aspartates can be employed, as can many other synthetic or natural polymeric materials bearing reactive functional groups. In addition, carbohydrates, yeasts, polysaccharides or any other substance that can be used as an immunological carrier can be conjugated to the hapten to produce an immunogen.

The Structure of the Tracers

The possible variations in the structure of the tracers of the invention are even greater than the possible variations in the structure of the haptens thereof. The tracers of the present invention have the general structural formula shown in Figure 2. In a

M 30·06·87

preferred form of the invention, the tracer has the structural formula shown in Figure 5.

The tracer is a 5-HIAA derivative that is linked to a fluorescein derivative by, for example, an amido, amidino, triazinylamino, carbamido, thiocarbamido, carbamoyl, thiocarbamoyl, or sulfonylcarbamoyl group, as shown in Figure 4. The tracers are prepared by linking the appropriate fluorescein derivative to a 5-HIAA derivative containing an amino, carboxylic acid, sulfonic acid, mercapto, hydroxy, imidate, hydrazide, isocyanate, thioisocyanate, chloroformate, chlorothioformate, carboxylic chloride, chlorosulfonylcarbamoyl, or the like group, as will be discussed in the context of the synthetic method and the examples.

By way of example, any of the following fluorescein derivatives can be used:

| | |
|---|---|
| $Fl-CH_2-NH_2$ | aminomethyl fluorescein |
| $Fl-NH_2$ | fluorescein amine |
| $Fl-CO_2H$ | α-iodacetamidofluorescein |
| $Fl-NHCOCH_2I$ | α-bromoacetamidofluorescein |
| 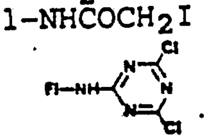 | 2, 4-dichloro-1,3, 5-triazin-2-ylamino-fluorescein (DTAF) |
| 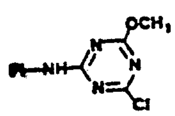 | 4-chloro-6- methoxy-1,3,5-triazin-2-ylamino-fluorescein |

The Antibodies

The antibodies of the present invention were prepared by eliciting a response in rabbits to the immunogens described supra. The immunogen is administered to animals or in vitro cultures of immunocompetent cells by a series of inoculations, in a manner well known to those skilled in the art. It should be understood that although rabbits were the preferred immune host to 5-HIAA immunogens in the

experiments detailed herein, any in vivo or in vitro host capable of producing antibodies to the structures herein outlined may be employed.

The Synthesis of Immunogens

The immunogens of the present invention are made by coupling a hapten, such as shown by the general structure of Figure 2 when Z is aldehyde, amino, hydroxy, chloroformate, iodoacetonyl or iodoacetyl to a poly(amino acid) or other immunologically active carrier. The poly(amino acid) or other carrier moiety can be linked to the hapten by a carbamate, amido, amino, thioether, ether linkage. In a preferred embodiment, the poly(amino acid) is thyroglobulin (TGB) or keyhole limpet hemocyanin (KLH) and the hapten has the structure shown in Figure 2 where Z'=H, R-Z=H. These reactants are preferably coupled under conditions normally used to form aminomethyl links by Mannich reaction; such conditions are well known to those skilled in the art.

The Synthesis of the Tracers

The tracers of the present invention are made by coupling a fluorescein moiety, on a derivative of fluorescein to a general structure shown in Figure 2 wherein R'=-Z-Y and $R^2$=H, wherein Z is alkyl, alkene chain or branched including up to 10 heteroatoms and having a total of from 0-20 carbon and heteroatoms arranged as a straight or branched chain and Y is $CO_2H$, CHO, $SO_3H$, CN, COCl, OH or $NH_2$ or R'= H and $R^2$=$NH_2$ or Z-Y, wherein Z is alkyl, alkene, chain or branched including up to 10 heteroatoms and having a total of from 0-20 carbon and hetero atoms arranged as a straight or branched chain and Y is $CO_2H$, CHO, $SO_3H$, CN, COCl, OH or $NH_2$.

The fluorescein moiety can be linked to the amino, carboxyl, aldehyde, acid chloride, imidate or alkoxy functional group by an amide, amine, a urea, thiourea, a carbamate, a thiocarbamate, triarylamino or sulfonylcarbamate linkage as shown in Figure 4. In the presently preferred embodiment, the fluorescein derivative is aminomethylfluorescein and this is coupled to a precursor shown in Figure 2a R'=H, with protected carboxylic functionality at 3-position as an methylester the appropriate carboxyalkyl or carboxy-aminoalkyl indole is coupled to aminomethylfluorescein by first forming the active ester with carboxylic group in 4-position. The preferred active ester is N-hydroxysucciniimide active ester and the preferred method is via N-N'-dicyclohexylcarbodiimide activation in anhydrous DMF. Other activating groups, such as acid chloride, 1-hydroxybenzotriazole, p-nitrophenol, or 2-ethyl-5-phenylisoxazolium-3'-sulfonate can be used; and other solvents such as tetrahydrofuran, dimethylsulfoxide, or hexamethylenephosphoramide. The reactants are preferable coupled under conditions for forming amide linkages, and it is not preferred that active ester procedures be used. Usable tracers can be prepared from a variety of 5-HIAA derivatives.

<u>The Assay</u>

The particular tracers, antibodies and additional reagents of the present invention have been found to measure both free 5-HIAA and free indoleacetic acid (IAA) levels in the urine when used without the specimen preparation method utilizing a phase extraction. In accordance with the analytical methods of the invention, i.e., the methods of determining 5-HIAA by a fluorescence immunoassay procedure using the tracer compounds and immunogens of the invention, an

extracted sample containing or suspected of containing 5-HIAA is intermixed with a biologically acceptable salt of the tracer, an antibody specific to both 5-HIAA and the tracer, and background quenching compound.

In a preferred embodiment of the invention, the reagents include pretreatment extraction reagents consisting of an organic extractant solvent, an acidic aqueous extractant and a reagent test pack consisting of fluorescein-labeled 5-HIAA (tracer), antibody specific for 5-HIAA and IAA and pretreatment reagents. Urinary 5-HIAA is first extracted from the urine into an organic phase and then subsequently back extracted into an aqueous phase. Examples of organic solvents that work well as extractant include dichloroethane and tertiary amyl alcohol. The aqueous extraction solution should be of low ionic strength, low pH, and electron-conducting.

This aqueous phase is then intermixed with antibody provided using the immunogen described above. Free 5-HIAA competes with the fluorescent tracer for limited antibody sites, resulting in the formation of complexes. By maintaining constant the concentration of tracer and antibody, one can assure that the ratio of 5-HIAA-antibody complex to tracer-antibody complex that is formed will be directly proportional to the total amount of 5-HIAA in the sample.

The results can be quantified in terms of net millipolarization units, span (in millipolarization units) and relative intensity. The measurement of millipolarization units indicates the maximum polarization when a maximum amount of the tracer is bound to the antibody in the absence of an 5-HIAA. The higher the net millipolarization units, the better the binding of the tracer to the antibody. The span is an indication of the difference between the net millipolarizations at the points of maximum and minimum

amount of tracer bound to the antibody. A larger span provides for a better numerical analysis of data. The intensity is a measure of the strength of the signal above background. Thus, a higher intensity will give a more accurate measurement. The intensity is determined at about 1800 intensity units (with the TDx(R) instrument gain set at 40) for the preferred tracers of the invention, as the sum of the vertically polarized intensity. The intensity of the tracer signal can range from about one to forty times the background noise, depending upon the concentration of the tracer and other assay variables.

The preferred method of the improved assay of the present invention will now be discussed in detail. The assay is a "homogenous assay", which means that the end polarization readings are taken from a solution in which bound tracer is not separated from unbound tracer. This is a distinct advantage over heterogenous immunoassay procedures, wherein the bound tracer must be separated from the unbound tracer before a reading can be taken.

The pH which the method of the present invention is practiced must be sufficient to allow the fluorescein moiety of the tracers to exist in their open form. The pH may range from about 5 to 10, more preferably in the range of from about 6 to 9, and most desirably from about 7.0 to 7.5. Relatively small changes in pH radically affect the intensity and special characteristics of fluorescence.

Various buffers may be used to achieve and maintain the pH of the homogenous immunoassay of the present invention during the assay procedure. Representative buffers include borate, citrate, phosphate, carbonate, tris, barbital and the like. The particular buffer employed is not critical to the

present invention, but the tris and phosphate buffers are preferred. The cation portion of the buffer will generally determine the cation portion of the tracer salt in solution.

The reagents for the fluorescence polarization assay of the present invention comprise antibody specific for 5-HIAA and tracer. Additionally, solutions including 5-HIAA pretreatment solutions, a dilution buffer, 5-HIAA calibrators and 5-HIAA controls are desirably prepared. Typical solutions of these reagents, some of which are described herein, are commercially available in assay "kits" from Abbott Laboratories, Abbott Park, Illinois.

All percentages expressed herein are weight/volume unless otherwise indicated. In the tracer formulation presently preferred, the preferred tracer compound is added to 98.5% dimethylformamide (volume/volume), 1.1% 12N hydrochloric acid (volume/volume), 0.1% thiourea and 0.38% potassium chloride solution (6.3 g potassium chloride in 100 ml $H_2O$) to yield about 1800 intensity units (instrument gain at 40). Various organic solvents can be used, together with various antioxidants and salts which are not only compatible but also (a) provide low pH and low pH stability; (b) provide low water content; and (c) are or are made electron conductive so as to be usable in a polarization analyzer. Preferred salts possessing the aforementioned properties are potassium and lithium chloride. Preferred antioxidants are ascorbic acid and N-acetyl cysteine. The antiserum formulation is comprised of rabbit serum diluted with 30% glycerol (volume/volume) and 0.03% sodium azide added to the common TDx(R) buffer used in the instrument.

The background quenching formulation is comprised of 50% sodium iodide in 0.1 M tris buffer.

Various metal salts and/or proteins may be used for background fluorescence reduction; examples include potassium or sodium iodide, riboflavin binding protein and the like. Calibrators are comprised of 5-HIAA in an acidic (pH 2.20) artificial urine matrix consisting of various salts, urea, creatinine, sodium azide, hydrochloric acid, which can be prepared by those skilled in the art, and novel use of antioxidants in the present invention which consists of 0.5% L-ascorbic acid and 0.5% N-acetyl-l-cysteine. Calibrator concentrations are 0.0, 2.5, 5.0, 8.0, 12.0 and 20 mcg/ml. Controls are comprised of 5-HIAA in the same artificial urine matrix as above at the concentrations of 3.5, 6.5 and 10 mcg/ml. All are purged and sealed under nitrogen or other nonoxygen gases such as argon or carbon dioxide.

The specimen pretreatment phase extractant necessary for reduction of specimen background intensity, interference and cross-reacting metabolites has been used in an assay using high performance liquid chromatography-electrochemical detection (HPLC-ECD) to reduce coeluting peaks (but not IAA, for IAA is not oxidized) and to reduce contamination of electrodes and columns. The present invention employs the phase extraction for the novel use of reduction of metabolites such as IAA, and for reduction of background fluorescence and interfering compounds. The reactants consist of an organic extractant solution and acid extractant solution. The organic extractant is comprised of 66% (volume/volume) dichloroethane and 33% (volume/volume) tertiary amyl alcohol. In addition, it can consist of various organic solvents known to those skilled in the art, such as hexane, heptane, dichloromethane, chloroform or ethylacetate, or mixtures thereof, either alone or in combination with various alcohols, such as tertiary amyl alcohol. The acid

extractant may be any aqueous solution, but preferably a low ionic strength aqueous solution that is electron conductive and has a low pH. Preferably the acid extractant is comprised of 0.01 M phosphoric acid in water. The use of the specimen phase extraction lowers the ratio of IAA amount to 5-HIAA amount resulting in 80% to 90% reduction in IAA measured. The amount of IAA excluded depends upon factors such as: a) specimen pH, where a low pH such as 3.5-1.0 favors a higher recovery ratio of 5-HIAA and a high pH such as 6-10 favors a high recovery ratio of IAA; b) organic solvents, where more polar solvents favor recovery of 5HIAA and less polar solvents favor recovery of IAA; c) volumes of sample, organic solvent and aqueous extractant solution; and (d) ionic strength of sample, organic solvent and aqueous extractant solution. In addition, the use of the specimen phase extraction reduces interfering compounds which could possibly increase background fluorescence resulting in inaccurate readings.

The preferred procedure is especially designed to be used in conjunction with the Abbott TDx$^{(R)}$ polarization analyzer available from Abbott Laboratories, Irving, Texas. Two hundred microliters of urine are required. The unknown samples must first be acidified within a pH range of 3.5 to 1.5. The preferred procedure is to add 6N hydrochloric acid until the pH of the urine is within the range of 3.5 to 1.5. The calibrators and controls are already acidified. The calibrators, controls or unknown samples are accurately pipetted (200 microliters) into a microcentrifuge tube, followed by an accurate volume of the organic extractant solution (200 microliters). This is mixed vigorously, preferably on a Vortex mixer and then centrifuged sufficiently until the two phases separate completely.

An accurate volume (100 microliters) of the bottom organic extract is removed and pipetted into a second microcentrifuge tube into which an accurate volume (200 microliters) of acid extractant has been pipetted. Volumes indicated are the preferred volumes, but any volume multiple of the solutions' ratios may be used. This second microcentrifuge tube is mixed vigorously and then centrifuged sufficiently until the two phases separate completely. At least 100 microliters of the top aqueous layer of the second microcentrifuge tube is transferred to the sample well of the TDx(R) sample cartridge. If a TDx(R) 5-HIAA (urine) assay kit is being used with the TDx(R) analyzer, the sample cartridges are placed directly into a sample carousel, the caps from each of the three reagent containers in the kit are removed and the reagent pack is placed into its designated well inside the TDx(R) analyzer. The instrument "run" button is pressed and from this point, the procedure is fully automated.

If a manual assay is being performed, then the sample is mixed with the pretreatment solution, the antibody and the dilution buffer and a background reading is taken. The tracer is then mixed with the assay. After incubation, a fluorescence polarization reading is taken.

The fluorescence polarization value of each calibrator, control or sample is determined and is printed on the output tape of an instrument such as the Abbott TDx(R) polarization analyzer. A standard curve is generated in the instrument by plotting the polarization of each calibrator versus its concentration using nonlinear regression analysis. The concentration of each control or sample is read off the stored calibration curve and printed on the output tape.

With respect to the foregoing preferred procedure, it should be noted that the tracer, antibody, pretreatment solution, calibrators, and controls should be stored between about 2 and about 8 degrees C, while the dilution buffer should be stored at ambient temperature. A standard curve and controls should be run every two weeks or whenever controls fall out of their designated ranges, with each calibrator and control run in duplicate. Controls should be run daily and all samples can be run in replicates if so desired.

It should be understood that the foregoing detailed description and the following examples are intended to be illustrative, but not limiting, with respect to the scope of the present invention. Various modifications will become apparent to one skilled in the art, and thus it is intended that the scope of the invention be defined solely by the claims and legal equivalents thereof.

## Examples

Examples 1 through 32 describe experiments that were performed in accordance with the concepts of the present invention.

### Example 1

### Coupling of 5-hydroxyindole-3-acetic acid to thyroglobulin (TGB)

One g of TGB was dissolved in 27 ml of water. To this solution was added a solution of 34.7 mg of 5-hydroxyindole-3-acetic acid in 4.5 ml of water and 2.4 ml of 3N sodium acetate and 7.3 ml of 7.5% formaldehyde. Reaction mixture was stirred at room temperature for 20 hours. The resulting solution was dialyzed exhaustively with water and lyophilized to give the desired conjugate, shown in Figure 10.

Example 2

Coupling of 5-hydroxyindole-3-acetic acid to keyhole limpet hemocyanin (KLH)

One g of KLH was dissolved in 27 ml of water. To this solution was added a solution of 34.7 mg of 5-hydroxyindole-3-acetic acid in 4.5 ml of water and 2.4 ml of 3N sodium acetate and 7.3 ml of 7.5% formaldehyde. Reaction mixture was stirred at room temperature for 20 hours. The resulting solution was dialyzed exhaustively with water and lyophilized to give the desired conjugate, shown in Figure 11.

Example 3

5-hydroxyindole-3-acetic acid methyl ester

500 mg of 5-hydroxyindole-3-acetic acid was dissolved in 5 ml of anhydrous methanol saturated with hydrogen chloride. After stirring for 1 hour at room temperature, reaction mixture was diluted with water, neutralized with 5% sodium bicarbonate and extracted with ethyl acetate. The organics were dried over magnesium sulfate and evaporated. Crude product was purified by column chromatography on silica gel with ethyl acetate:hexanes (50:150) as an eluent to afford 235 mg (88%) of desired ester, shown in Figure 12.

Example 4

5-hydroxy 4-(2-aminoethyl)-3-indoleacetic acid methyl ester

To 100 mg (0.48 mmol) of 5-hydroxy-3-indoleacetic acid methyl ester dissolved in 1 ml of ethanol was added 0.5 mL of 37% formaldehyde followed by 0.5 mL of 40% dimethylamine. After 2 minutes, reaction mixture was diluted with 5 mL of ethyl acetate and transferred to a separatory funnel. The organics were

washed with water, and dried over magnesium sulfate. Crude product was purified by column chromatography on silica gel using acetone as an eluent. Yield was 85 mg of colorless crystals(mp. 110°C), shown in Figure 13.

Example 5

5-hydroxy-3-carbomethoxymethyl-4-indolymethyl trimethyl-ammonium iodide

To 356 mg (1.35 mmol) of 5-hydroxy-4-(dimethylaminomethyl)-3-indoleacetic acid methyl ester in 30 mL of anhydrous ethyl ester was added 3 mL of methyl iodide. After 24 hours precipitated solid was filtered off to afford 547 mg of desired salt, shown in Figure 14.

Example 6

5-hydroxy-4-(2-nitroethyl)-3-indoleacetic acid methyl ester

To 547 mg (1.35) of (5-hydroxy-3-carbomethoxymethyl-4-indolemethyl)-trimethylammonium iodide in 5 mL of anhydrous nitromethane was added 54 mg of finely pulverized sodium hydroxide. Reaction mixture was refluxed for 4 hours under nitrogen atmosphere and subsequently transferred to a separatory funnel containing 40 ml of saturated solution of boric acid. Reaction mixture was extracted with chloroform (total 100 ml) and dried over magnesium sulfate. Crude product was purified by column chromatography on silica gel with ethyl acetate:hexanes (1:1) as an eluent to afford 114 mg (33%) of desired product, shown in Figure 15.

Example 7

5-hydroxy-4-(2-aminoethyl)-3-indoleacetic acid methyl ester

Solution of 50 mg of 5-hydroxy-4-(2-nitroethyl)-3-indoleacetic acid methyl ester in 0.5 $cm^3$ of ethyl

acetate was added to 5 mg of prehydrogenated platinum oxide in 1 $cm^3$ of ethyl acetate:ethanol (1:1). Reaction mixture was stirred under the hydrogen balloon and monitored by TLC. After 3.5 hours, reaction mixture was filtered from catalyst, solvent was removed and residue purified by PTLC on silica gel using chloroform-methanol-acetic acid 90:10:5 as an eluent to afford 10 mg of colorless solid, shown in Figure 16. Mass spectrum (70 eV) relative intensity 248 ($M^+$=248), 231 (50), 219(100).

Example 8

5-hydroxy-4-(N-carbobenzoxyglycylamino)-3-indole active acid methyl ester

54 mg (0.21 mmol) of 5-hydroxy-4-(2-aminoethyl)-3-indoleacetic acid methyl ester and 60 mg (0.18 mmol) of N-carbobenzoxyglycine-p-nitrophenyl ester was dissolved in 3 mL of DMF. Reaction mixture was stirred for 15 hours at room temperature, and rotoevaporated *in vacuo*. The residue was dissolved in ethyl acetate and purified by column chromatography on silica gel using acetone hexanes 2:1 as an eluent (Rf=0.52) to afford 60 mg of pure desired material, shown in Figure 17.

Example 9

5-hydroxy-4-(glycylaminoethyl)-3-indole acetic acid methyl ester

60 mg of compound prepared in Example 8, *supra*, was hydrogenated in 4 mL of ethanol and 5 mg of 10% Pd/C under 40 psi. After 2.5 hours all starting material was consumed (TLC control). Reaction mixture was filtered through Celite, washed with ethanol and concentrated *in vacuo*. Yield was 30.1 mg (74%) of desired product, shown in Figure 18.

Example 10

5-carboxymethyloxy indole-3-acetic acid methyl ester

205 mg (1 mmol) of 5-hydroxyindole-3-acetic acid methyl ester was dissolved in 5 mL of acetone and two portions of 552 mg each of finely pulverized potassium carbomate were added followed by 332 mL of tertbutyl bromoacetate. Reaction mixture was refluxed for 4 hours, filtered from inorganics and organics filtrate was evaporated. The oily residue was purified by column chromatography on silica gel with ethyl acetate-hexanes to afford desired t-butyl-methyl esters. Yield was 91%. This product was dissolved in 3 mL of 1:2 mixture of trifluoroacetic acid:methylene chloride. After 1 hour at room temperature, the reaction mixture was evaporated to dryness to give the desired product, 5-carboxymethyloxyindole-3-acetic acid methyl ester (89% yield), shown in Figure 19.

Example 11

5-hydroxy-4-(N-carboxymethyl, N-methyl)-aminomethyl-indole-3-acetic acid methyl ester

To a 100 mL flask was added 254.3 mg (1 equiv) of paraformaldehyde, 770 mg of N-methylaminoacetic acid and 20mL of dioxane. Reaction mixture was stirred in oil bath (50°C) for 30 minutes and cooled to room temperature. 1738 mg of 5-hydroxyindole-3-acetic acid methyl ester was added and 866 mg of triethylamine in 20 ml of dioxane. Reaction mixture was stirred for 5 days and after removing solvent, purified by chromatography on silica gel to afford 492 mg of desired material, shown in Figure 20.

11.20.06.87

Example 12

Coupling of 5-hydroxy-4-(N-carboxymethyl, N-methyl)aminomethyl indole-3-acetic acid methyl ester to aminomethylfluorescein

5-hydroxy-4-(N-carboxymethyl, N-methyl)-aminomethyl indole-3-acetic acid methyl ester was dissolved in 13 mL of dimethylformamide and 197 mg of N-hydroxysucciniimide followed by 286 mg of 1,3-dicyclohexylcarbodiimide. Reaction mixture was stirred for 8 hours and then filtered into the solution of 345 mg aminomethylfluorescein and triethylamine 138 mg in 9 mL DMF. The reaction mixture was stirred at room temperature for 14 hours. Crude product was purified by column chromatography on silica gel. Yield was 62% of desired product (hydrolysis of ester). This product was dissolved in 4 mL of methanol and 4 mL of 1N sodium hydroxide was added and reaction mixture stirred for 1 hour at room temperature. The reaction mixture was neutralized with 1N hydrodilonic acid and rotevaporated in vacuo. Yellow solid, shown in Figure 6, was purified by PTLC ($RPC_{18}$ with $H_2O$-MeOH-HOAc (60:60:0.5; Rf 0.5).

Example 13

Coupling of-5-carboxymethyloxyindole-3-acetic acid methyl ester with aminomethylfluorescein

13 mg of 5-carboxymethyloxyindole-3-acetic acid methyl ester was dissolved in 0.2 mL of anhydrous dimethylformamide and to this solution was added 10 mg of dicyclohexylcarbodiimide dissolved in 0.2 mL of DMF followed by solution of 6 mg N-hydroxysucciniimide in 0.2 mL of DMF. Mixture was stirred for 0.5 hours and 22 mg of aminomethylfluorescein was added. After 12 hours reaction mixture was evaporation in vacuo and product purified by chromatography on silica gel using ethyl acetate:acetic acid (100:0.2) as an eluent. Product was

hydrolyzed by 10% sodium hydroxide for 0.5 hours and purified by chromatography to yield desired tracer, shown in Figure 7.

Example 14

Coupling of 5-hydroxy-4-(2-aminoethyl, 3-indoleacetic acid methyl ester to 5-carboxyfluorescein active ester

To 35 mg of 5-hydroxy-4-(2-aminoethyl)-3-indole acetic acid methyl ester in 1 mL of DMF was added 68 mg of 5-carboxyfluorescein active ester (with N-hydroxysucciniimide). Reaction mixture was stirred for 12 hours and evaporated to dryness. Crude material was dissolved in 3 mL of 10% sodium hydroxide and after 0.5 hours at room temperature acidified with acetic acid. Desired product, shown in Figure 8, was obtained after purification by TLC.

Example 15

Coupling of 5-hydroxy-4-(glycylaminoethyl)-3-indole acetic acid methyl ester to carboxyfluorescein

This compound, shown in Figure 9, was prepared from 5-hydroxy-4-(glycylaminoethyl)-3-indole acetic acid methyl ester to carboxyfluorescein essentially as in Example 15.

Example 16

5-bromoindole 3-acetic acid methyl ester

2 g (7.87 mmol) of 5-bromoindole 3-acetic acid was dissolved in 16 mL of anhydrous methanol saturated with hydrogen chloride. After stirring for 1 hour at room tempeerature, the reaction mixture was diluted with water, neutralized with saturated sodium bicarbonate and extracted with chloroform. The organics were dried over magnesium sulfate and evaporated to yield 1.7 g (81%) of desired material as a white solid, shown in Figure 21.

Example 17

N-alkylation of 5-bromoindole 3-acetic acid methyl ester with bromoacetaldehyde diethyl acetal of 5-bromo-N-formyl methyl-3-carbomethoxymethylindole

Solution of 400 mg (1.49 mmol) of 5-bromoindole 3-acetic acid methyl ester in 4.7 mL of anhydrous dimethylformamide was reacted for 30 minutes with 89,.5 mg of sodium hydride (60% oil suspension). 545.5 mg of bromoacetaldehyde diethyl acetal was added, and the reaction ixture was refluxed under the nitrogen for 6 hours. Dimethylformamide was removed under vacuum, and the residue was extracted with ethyl ether. Ethyl ether was evaporated and the residue purified by column chromatography on silica gel with 40% ethyl acetate-hexanes to yield 37% of desired material, shown in Figure 22.

Example 18

Diethyl acetal of 5-bromo-N-formylmethyl 3-carboxymethyl indole

193 mg of diethyl acetal of 5-bromo-N-formyl methyl-3-carboxymethylindole was dissolved in a 5 mL mixture of 10% aqueous sodium hydroxide:tetrahydrofuran:methanol (1:1:1). The reaction mixture was stirred at room temperature for 1 hour and next diluted with water, acidified to pH 4 with dilute hydrochloric acid and extracted with ethyl ether. Organics were dried over magnesium sulfate and evaporated under vacuum to yield the desired product, shown in Figure 23.

Example 19

5-bromo-N-formylmethyl 3-carboxy-methyl indole

80 mg of diethylacetal of 5-bromo-N formylmethyl 3-carboxy-methyl indole was dissolved in 16

mL of acetone:water mixture (5:1) and 2 g of cation exchange resin AG 50W-X8 was added. Reaction mixture was refluxed under nitrogen for 10 hours, then filtered off. Solvent was removed under vacuum to yield the desired product, shown in Figure 24.

Example 20

Coupling of 5-bromo-N-formylmethyl 3-carboxymethyl indole to bovine serum albumin (BSA)

120 mg of BSA was dissolved in 4 mL of water. To this solution was added a solution of 21 mg of 5-bromo-N-formylmethyl 3-carboxymethyl indole in 3.5 mL of dioxane. The pH was adjusted to pH 6 withhydrochloric acid, and the reaction mixture was stirred for 2 hours, followed by the addition of 30 mg of sodium cyanoborohydride. After additional stirring for 16 hours, the pH was adjusted to pH 8.5 with a saturated solution of sodium bicarbonate and was dialyzed exhaustively with water and lyophilized to give the desired conjugate, shown in Figure 25.

Example 21

5-bromo-N-γ-chloropropyl 3-carbomethoxymethyl indole

A solution of 300 mg (1.12 mmol) of 5-bromoindole 3-acetic acid methyl ester in 4 mL of anhydrous dimethylformamide was reacted for 30 minutes with 67 mg (1.5 equiv) of sodium hydride (60% oil suspension). 412 mg (1.8 equiv) of 1-chloro-3-iodopropane was added, and the reaction mixture was stirred at room temperature for 12 hours. Dimethylformamide was removed under vacuum and the residue was extracted with ethyl ether. Ethyl ether was evaporated and the residue purified by column chromatography on silica gel to yield the desired material, shown in Figure 26.

## Example 22

## 5-bromo-N-γ-iodopropyl-3-carbomethoxymethyl indole

To 112.7 mg of 5-bromo-N-γ-chloropropyl-3-carbomethoxymethyl indole dissolved in 6 mL of butanone was added 147 mg of sodium iodide. The reaction mixture was refluxed for 16 hours and solvent was removed under vacuum. The residue was extracted with ethyl ether, washed with water and brine, and the organics were dried over magnesium sulfate and evaporated. The crude product was purified by preparative thin-layer chromatography on 1 mm silica gel plates with 35% ethyl acetate:hexanes as an eluent to afford 156 mg of desired product, shown in Figure 27.

## Example 23

## 5-bromo-N-γ-nitropropyl-3-carbomethoxymethyl indole

To 137 mg of 5-bromo-N-γ-iodopropyl-3-carbomethoxymethyl indole dissolved in 10 mL of anhydrous ethyl ether was added 147.5 mg of silver nitrite. The reaction mixture was refluxed for 17 hours. The reaction mixture was then filtered from insoluble inorganics and the filtrate evaporated. Crude product was purified by column chromatography on silica gel using 35:65 ethyl acetate:hexanes as an eluent. Yield was 87 mg (77%) of desired product, shown in Figure 28. Mass spectrum: ($M^+$ 354).

## Example 24

## 5-bromo-N-γ-aminopropyl-3-carbomethoxymethyl indole

To 87 mg of 5-bromo-N-γ-nitropropyl-3-carbomethoxymethyl indole dissolved in 5 mL ethyl acetate:anhydrous ethanole (7:3) was added 5 mg of platinum oxide and the mixture was hydrogenated with hydrogen at atmosphereic pressure for 24 hours. The

reaction mixture was separated from inorganics and the solvent removed by evaporation <u>in vacuo</u> to yield 70 mg of desired product, shown in Figure 29. Mass spectrum: $(M^{+}324)$.

## Example 25

### Coupling of 5-bromo-N-$\gamma$-aminopropyl-3-carbomethoxymethyl indole to 6-carboxyfluorescein-N-hydroxysuccinimide ester

To 20 mg of 5-bromo-N-$\gamma$-aminopropyl-3-carbomethoxymethyl indole in 0.2 mL of dimethylformamide was added 15 mg of 6-carboxyfluorescein-N-hydroxysuccinimide ester. The reaction mixture was stirred at room temperature for 15 hours and evaporated to dryness. Crude product was purified by preparative thin-layer chromatography on Whatman $PLKC_{18}$ plates using water:methanol:acetic acid (40:60:0.5). The yield was 14.4 mg (81%). Mass spectrum: $M^{+}$=(623, M+H).

This product was dissolved in 1 mL of methanol and 400 uL of 10% sodium hydroxide was added to hydrolyze selectively the 3-carbomethoxymethyl group of indole to carboxylic acid. After 30 minutes, the reaction mixture was acidified to pH 6 with glacial acetic acid and evaporated to dryness. Crude product was purified by chromatography. See Figure 30.

## Example 26

This example illustrates th accuracy of the method of the present invention vis-a-vis the primary method currently in use, colorimetric assay.

<u>TDx vs. Colorimetric (N-102)</u>

Intercept = 1.43
Slope = 0.91
Coefficient of correlation = 0.999
Range = 0.48 to 672 ug/ml

## Example 27

This example illustrates the accuracy of the method of the present invention vis-a-vis high-pressure liquid chromatography with electro-chemical detection (HPLC-ECD).

TDx vs. HPLC-ECD(N=129)

Intercept = 0.28
Slope = 1.02
Coefficient of correlation = 0.976
Range = 0.39 to 133.6 ug/ml

## Example 28

This example, wherein to 5 urine samples are added 5-HIAA and the percentage of the addition recovered in each instance is determined, further assesses the accuracy of the assay of the present invention.

To an aliquot of each acidified urine sample was added a concentrated stock solution of 5-HIAA to levels of 0, 5, 8 and 12 ug/ml 5-HIAA. Specimen phase extraction pretreatment was performed as described in the Summary of the Invention.

The TDx analyzer was calibrated with artificial urine matrix calibrators at 0, 2.5, 5, 8, 12 and 20 ug/ml which were treated in the same fashion as the samples. The standard curve was stored in the instrument and samples were read off the curve.

The percentage of added 5-HIAA recovered was determined as follows: endogenous 5-HIAA (0 ug/ml added) was subtracted from the urine spike levels and divided by the target value of the 5-HIAA added.

Adjusted concentration = concentration measured minus endogenous level.

$$\%\ \text{recovery} = \frac{\text{Adjusted concentration}}{\text{concentration of 5-HIAA added}} \times 100$$

The results are shown in the following table:

| Added Concentration (N=5) | % Recovery |
|---|---|
| 5.0 | 97.4 |
| 8.0 | 97.8 |
| 12.0 | 97.4 |

Example 29

This example illustrates the stabilizing function of the calibrator and control matrix.

On day 0 calibrators were run in duplicate samples. Every working day that followed the vials were opened for one hour, protected from light at room temperature and then sealed and stored at 2-8°C. The samples were then tested once per week for five weeks.

| Calib. Conc. | | | mP Values | | | |
|---|---|---|---|---|---|---|
| Timept. | 0ug/ml | 2.5ug/ml | 5ug/ml | 8ug/ml | 12ug/ml | 20ug/ml |
| Day 0 | 296.7 | 179.4 | 164.8 | 152.4 | 140.2 | 123.9 |
| Wk 1 | 210.0 | 182.6 | 166.8 | 155.2 | 143.2 | 125.5 |
| Wk 2 | 210.5 | 182.9 | 168.0 | 154.8 | 143.4 | 125.9 |
| Wk 3 | 207.3 | 181.8 | 166.0 | 153.4 | 141.4 | 125.4 |
| Wk 4 | 216.0 | 185.8 | 170.3 | 156.2 | 143.5 | 127.1 |
| Wk 5 | 211.5 | 181.9 | 165.4 | 153.0 | 139.9 | 124.9 |

Example 30

This example illustrates the stabilizing function of the tracer matrix:

On day 0 calibrators at 0 ug/ml and 20 ug/ml were run in duplicate samples for each storage condition of 2-8°C continuously, room temperature continuously and -20°C for 72 hours and then 2-8°C continuously. This was repeated at days 7 and 14.

2-8°C Continuous Storage

| | | mP Values | |
|---|---|---|---|
| | 20 ug/ml span | 0 ug/ml | 20 ug/ml |
| 104.4 | 247.9 | 143.5 | |
| Day 7 | 106.9 | 244.7 | 137.9 |
| Day 14 | 102.4 | 243.1 | 140.4 |

-20°C Freeze/Thaw Storage

| | 0-20ug/ml Span | mP Values 0ug/ml | 20ug/ml |
|---|---|---|---|
| Day 0 | 104.4 | 247.9 | 143.5 |
| Day 7 | 106.0 | 246.8 | 140.8 |
| Day 14 | 105.8 | 244.3 | 138.5 |

Room Temperature Continuous Storage

| | 0-20ug/ml Span | mP Values 0ug/ml | 20ug/ml |
|---|---|---|---|
| Day 0 | 104.4 | 247.9 | 143.5 |
| Day 7 | 103.7 | 247.4 | 136.7 |
| Day 14 | 108.4 | 246.5 | 138.1 |

Example 31

This example illustrates the development of desirable antisera in multiple rabbits with multiple immunogen preparations. Three rabbits were innoculated and boosted with three immunogen lots. Representative standard curves generated using antisera from each animal are shown below.

| Immunogen Lot # | 13372-43 | 12618-240 mP Values | 13372-251 |
|---|---|---|---|
| Animal # | 829 | 815 | 2197 |
| 0 ug/ml | 207.3 | 219.4 | 247.2 |
| 2.5ug/ml | 161.7 | 169.4 | 218.6 |
| 5.0ug/ml | 139.2 | 146.3 | 200.8 |
| 8.0ug/ml | 122.8 | 132.5 | 184.2 |
| 12.0ug/ml | 110.5 | 120.5 | 168.1 |
| 20.0ug/ml | 96.5 | 108.2 | 149.9 |
| Span | 111 | 111 | 98 |

Example 32

This example illustrates the reduction of both background fluorescence and indoleacetic acid measured when using the specimen pretreatment phase extraction protocol. Calibration curves were run, in duplicate, with and without the specimen phase extraction. Indoleacetic acid at 10 mcg/ml and 5 urines with high

background fluorescence were run, in duplicate, both extracted and unextracted against their respective calibration curves.

| | Specimens Unextracted | Specimens Extracted |
| --- | --- | --- |
| 10 mcg/ml indoleacetic acid | 3.1 mcg/ml | 0.61 mcg/ml |
| | Background Intensity | |
| Urine #1 | 5984 | 559 |
| Urine #2 | 20492 | 1138 |
| Urine #3 | 10337 | 707 |
| Urine #4 | 20445 | 1214 |
| Urine #5 | 20309 | 1136 |

CLAIMS:

1. A compound having the structure:

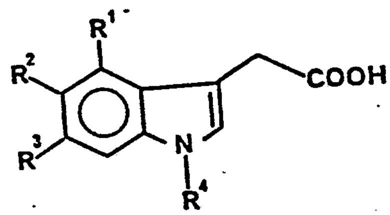

wherein:

$R_1$ is H or R-Z-Q or together with $R_2$ is a cyclic group having a total of from 4 to 8 carbon atoms and containing up to 4 heteroatoms;

$R_2$ is OH, Cl, F, Br, I, $OCH_3$, ORZQ, or together with $R_1$ or $R_3$ is a cyclic group having a total of from 4 to 8 carbon atoms and containing up to 4 heteroatoms;

$R_3$ is H or R-Z-Q or together with $R_2$ is a cyclic group having a total of from 4 to 8 carbon atoms and containing up to 4 heteroatoms;

$R_4$ is H or R-Z-Q;

Q is a poly (amino acid), a poly (amino acid) derivative or another immunologically active carrier, or fluorescein or a fluorescein derivative;

Z is CO, NH, $CH_2NH$ or CS when Q is fluorescein or a fluorescein derivative and is N, NH, $SO_2$, $PO_2$, PSO or a glucuronide moiety when Q is a poly (amino acid), a poly (amino acid) derivative or another immunologically active carrier; and

R is a linking group including up to 7 heteroatoms when Q is a poly (amino acid), a poly (amino acid) derivative or other immunologically active carrier and including up to 10 heteroatoms when Q is fluorescein or a fluorescein derivative, and having a total of from 0 to 20 carbon atoms and heteroatoms arranged in a straight or branched chain and containing up to two ring structures.

2. The compound of claim 1 wherein Q is a 4'-aminomethylfluorescein derivative.

3. A compound having the structure:

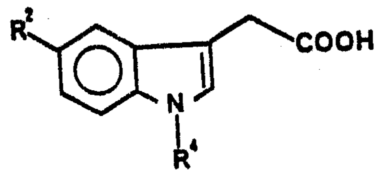

wherein:

$R_2$ is H, OH, Cl, F, Br, or I;

$R_4$ is R-Z-Q;

Q is a poly(amino acid), a poly(amino acid) derivative or another immunologically active acrrier, or fluorescein or a fluorescein derivative;

Z is CO, NH, $CH_2NH$ or CS when Q is fluorescein or a fluorescein derivative and is N, NH, $SO_2$, $PO_2$, PSO or a flucuronide moiety when Q is a poly(amino acid), a poly(amino acid) derivative or another immunologically active carrier; and

R is a linking group including up to 7 heteroatoms when Q is a poly(amino acid), a poly(amino acid) derivative or other immunologically active carrier and including up to 6 heteroatoms when Q is fluorescein or a fluorescein derivative, and having a total of from 0 to 10 carbon atoms and heteroatoms arranged in a straight or branched chain and containing up to two ring structures.

4. A method for making an immunogen comprising the step of coupling a compound of the formula:

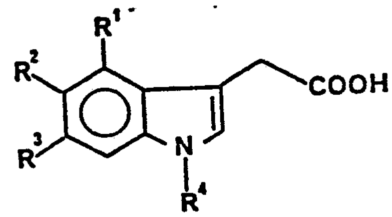

wherein:

$R_1$ is H or R-X or together with $R_2$ is a cyclic group having a total of from 4 to 8 carbon atoms and containing up to 4 heteroatoms;

$R_2$ is OH, Cl, F, Br, I, $OCH_3$, ORZQ, or together with $R_1$ or $R_3$ is a cyclic group having a total of from 4 to 8 carbon atoms and containing up to 4 heteroatoms;

$R_3$ is H or R-X or together with $R_2$ is a cyclic group having a total of from 4 to 8 carbon atoms and containing up to 4 heteroatoms;

$R_4$ is H or R-X;

Q is a poly (amino acid), a poly (amino acid) derivative or another immunologically active carrier, or fluorescein or a fluorescein derivative;

Z is CO, NH, $CH_2NH$ or CS when Q is fluorescein or a fluorescein derivative and is N, NH, $SO_2$, $PO_2$, PSO or a flucuronide moiety when Q is a poly (amino acid), a poly (amino acid) derivative or another immunologically active carrier; and

R is a linking group including up to 7 heteroatoms when Q is a poly (amino acid), a poly (amino acid) derivative or other immunologically active carrier and including up to 10 heteroatoms when Q is fluorescein or a or a fluorescein derivative, and having a total of from 0 to 20 carbon atoms and heteroatoms arranged in a straight or branched chain and containing up to two ring structures.

5. A method for making a tracer comprising the step of coupling a precursor of the formula:

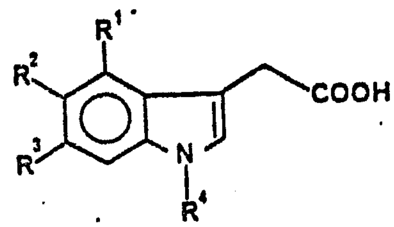

wherein:

$R_1$ is H or R-Y or is taken together with $R_2$ as a cyclic group having a total of from 4 to 8 carbon atoms and containing up to 4 heteroatoms;

$R_2$ is OH, Cl, F, Br, I, $OCH_3$, ORZQ, or is taken together with $R_1$ or $R_3$ as a cyclic group having a total of from 4 to 8 carbon atoms and containing up to 4 heteroatoms;

$R_3$ is H or R-Y or is taken together with $R_2$ as a cyclic group having a total of from 4 to 8 carbon atoms and containing up to 4 heteroatoms;

$R_4$ is H or R-Y;

Q is a poly (amino acid), a poly (amino acid) derivative or another immunologically active carrier, or fluorescein or a fluorescein derivative;

Z is CO, NH, $CH_2NH$ or CS when Q is fluorescein or a fluorescein derivative and is N, NH, $SO_2$, $PO_2$, PSO or a glucuronide moiety when Q is a poly (amino acid), a poly (amino acid) derivative or another immunologically active carrier; and

R is a linking group including up to 7 heteroatoms when Q is a poly (amino acid), a poly (amino acid) derivative or other immunologically active carrier and including up to 10 heteroatoms when Q is fluorescein or a fluorescein derivative, and having a total of from 0 to 20 carbon atoms and heteroatoms arranged in a straight or branched chain and containing up to two ring structures;

with fluorescein or a derivative of fluorescein.

6. A method for determining the 5-hydroxy-3-indoleacetic acid content of a fluid sample, which comprises the steps of:

(a) contacting the fluid sample with a 5-hydroxy-3-indoleacetic acid antiserum, a background quenching compound, and a compound of claim 1 capable of producing a detectable fluorescence polarization response to the presence of the 5-hydroxy-3-indoleacetic acid antiserum;

(b) passing plane polarized light through the resulting solution from step (a) to obtain a fluorescence polarization response; and

(c) detecting the fluorescence polarization response of the solution of step (b) as a measure of the 5-hydroxy-3-indoleacetic acid content of the sample.

7. The process of claim 6 wherein the background quenching compound is sodium iodide.

8. A composition providing a stabilizing matrix for a tracer, comprising:

(a) an organic solvent having a pH in the range of 1.5 to 2.5;

(b) an antioxidant that is stable and soluble in said organic solvent; and

(c) an electron-conductive compound.

9. The composition of claim 8 wherein the organic solvent is N,N-dimethylformamide, the antioxidant is thiourea, and the electron-conductive compound is potassium chloride in hydrochloric acid.

10. A method for preparing an artificial urine matrix for controls and calibrators used in a fluorescence polarization immunoassay, which comprises the steps of:

(a) purging an artifical urine composition with oxygen-free gas;

(b) adding to the purged artificial urine composition a combination of L-ascorbic acid and N-acetyl-L-cysteine; and

(c) sealing from oxygen the resultant stabilized composition.

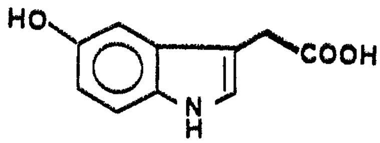

FIG. 1

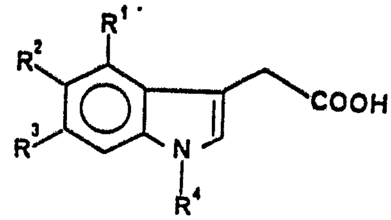

FIG. 2

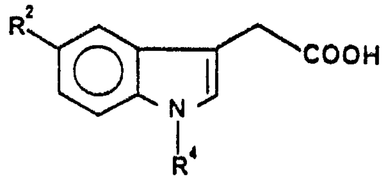

FIG. 3

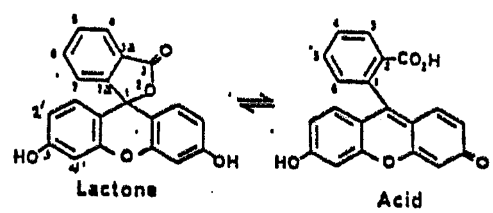


FIG. 4

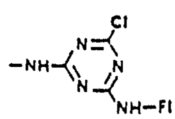 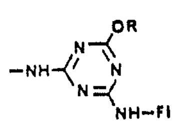, 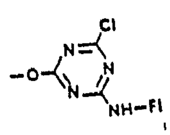

-NH-CO-Fl   -CO-NH-Fl   -C(NH)-NH-Fl

-NH-CO-NH-Fl   -NH-CS-NH-Fl   -O-CO-NH-Fl

-O-CS-NH-Fl   $-SO_2$-NH-Fl   -O-CO-NH-$SO_2$-NHFl

FIG. 5

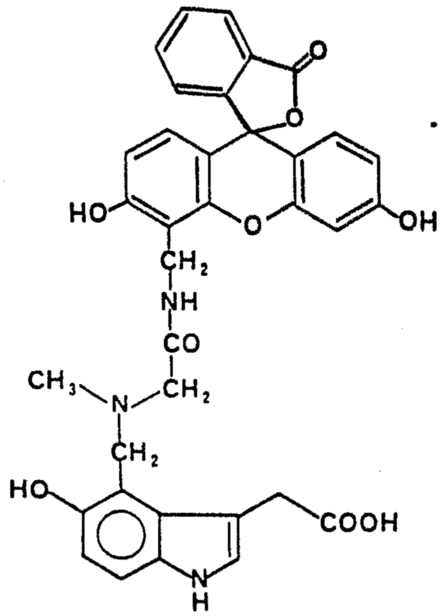

FIG. 6

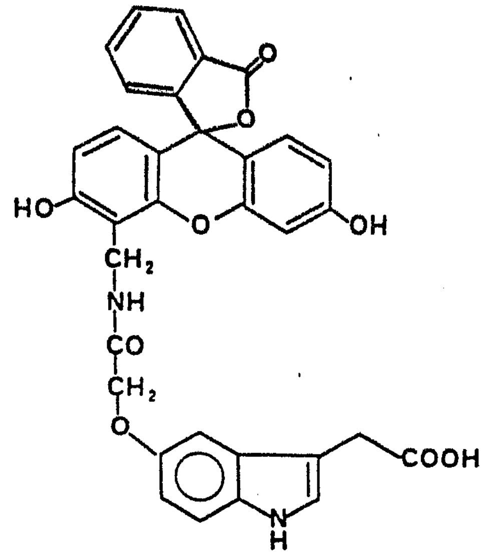

FIG. 7

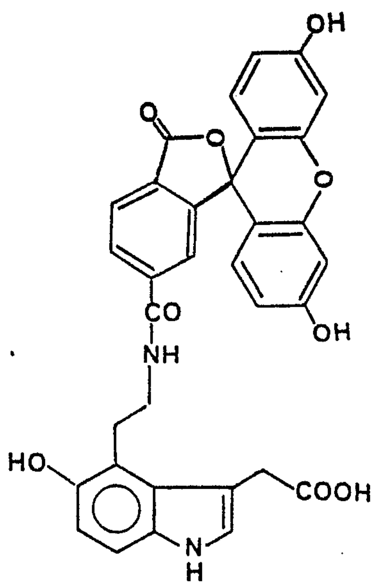

FIG. 8

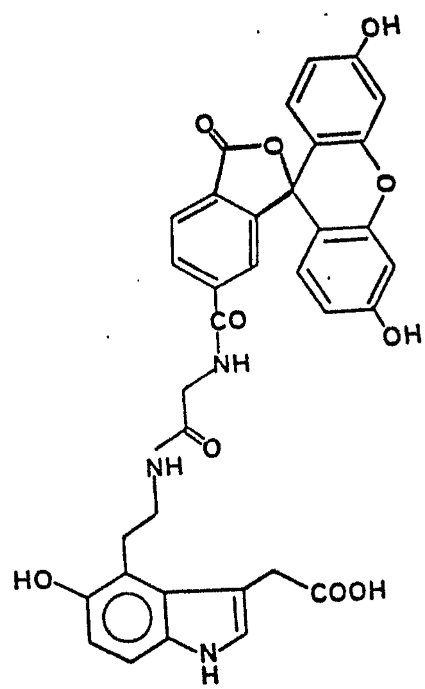

FIG. 9

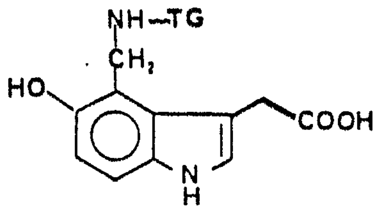

FIG. 10

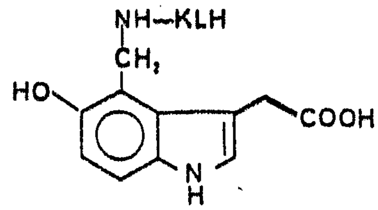

FIG. 11

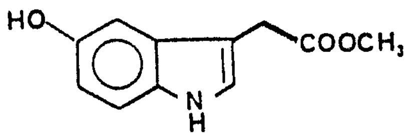

FIG. 12

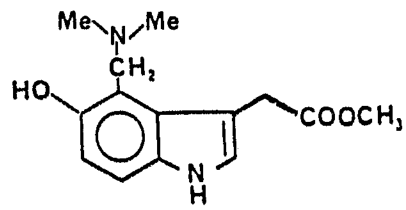

FIG. 13

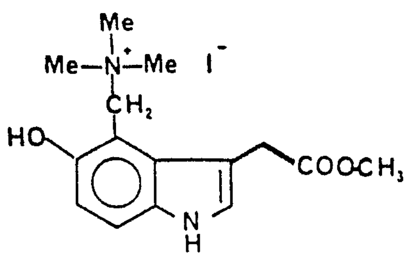

FIG. 14

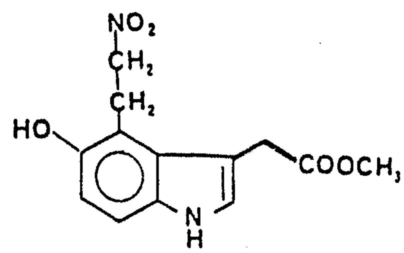

FIG. 15

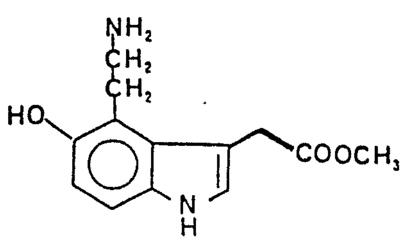

FIG. 16

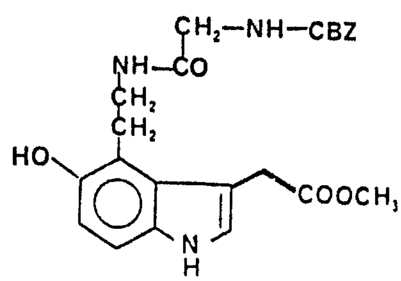

FIG. 17

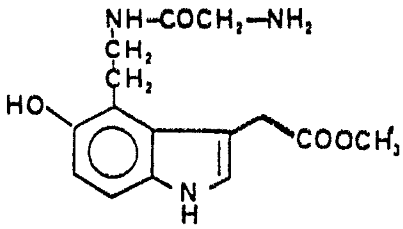

FIG. 18

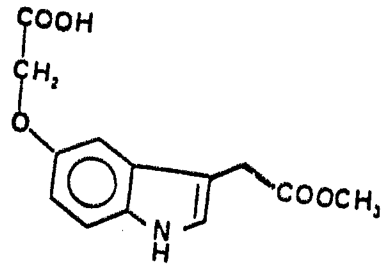

FIG. 19

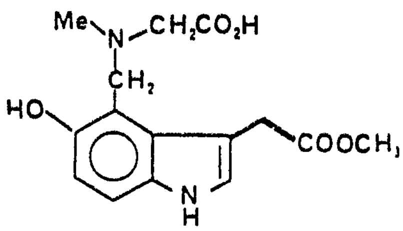

FIG. 20

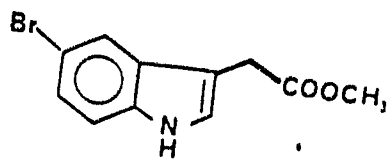

FIG. 21

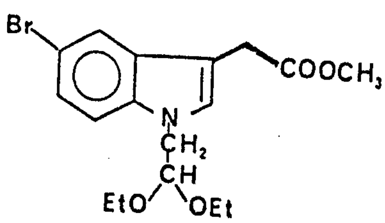

FIG. 22

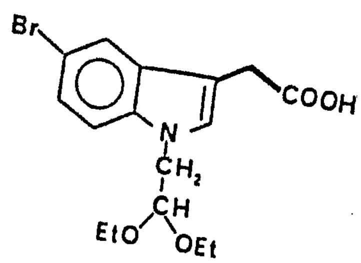

FIG. 23

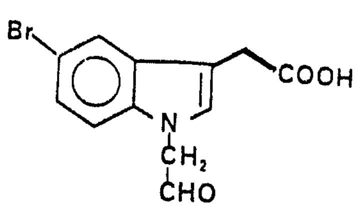

FIG. 24

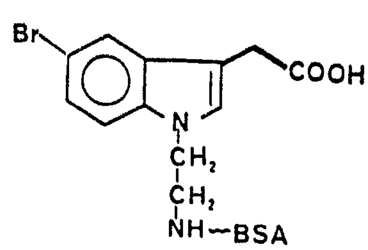

FIG. 25

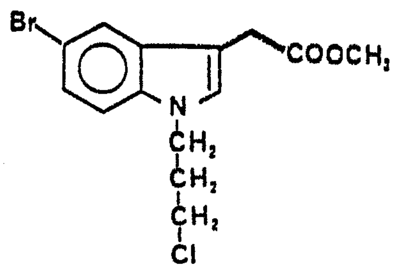

FIG. 26

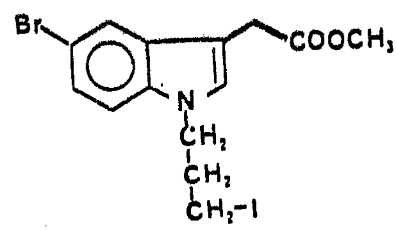

FIG. 27

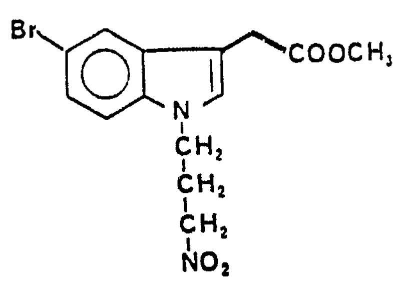

FIG. 28

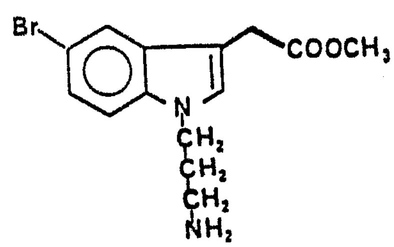

FIG. 29

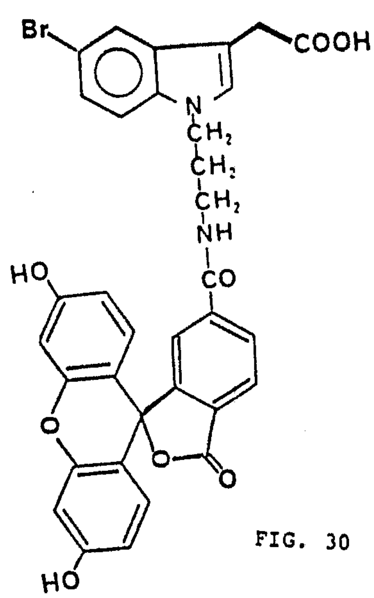

FIG. 30